# EUROPÄISCHE PATENTSCHRIFT

(11) **EP 3 154 474 B1**
(45) Veröffentlichungstag und Bekanntmachung des Hinweises auf die Patenterteilung: **10.11.2021**
(21) Anmeldenummer: 14816331.4
(22) Anmeldetag: 23.12.2014
(51) Int. Cl.: A61F 2/24, A61F 2/856

(54) **HERZKLAPPENPROTHESE ZUM PERKUTANEN ERSATZ EINER TRIKUSPIDALKLAPPE, SET UND SYSTEM MIT EINER DERARTIGEN HERZKLAPPENPROTHESE**
HEART VALVE PROSTHESIS FOR PERCUTANEOUS REPLACEMENT OF A TRICUSPID VALVE, SET AND SYSTEM COMPRISING A HEART VALVE PROSTHESIS OF SAID TYPE
PROTHÈSE DE VALVULE CARDIAQUE POUR LE REMPLACEMENT PERCUTANÉ DE LA VALVULE TRICUSPIDE, ENSEMBLE ET SYSTÈME COMPORTANT UNE TELLE PROTHÈSE DE VALVULE CARDIAQUE

(30) Priorität: 16.07.2014 WO PCT/EP2014/065280
(43) Veröffentlichungstag der Anmeldung: 19.04.2017
(73) Patentinhaber: P+F Products + Features GmbH, 1190 Wien (AT)
(72) Erfinder: FIGULLA, Hans, Reiner, 07749 Jena (DE); LAUTEN, Alexander, 14532 Kleinmachnow (DE)
(74) Vertreter: Manitz Finsterwald Patent- und Rechtsanwaltspartnerschaft mbB
(86) Internationale Anmeldenummer: PCT/EP2014/079219
(87) Internationale Veröffentlichungsnummer: WO 2016/008551

(56) Entgegenhaltungen:
- WO-A2-2014/086871
- DE-A1-102010 046 459
- US-A1- 2004 206 363
- US-A1- 2006 276 813
- US-A1- 2011 106 244

## Beschreibung

Die Erfindung betrifft eine Herzklappenprothese zum perkutanen Ersatz einer Trikuspidalklappe. Ferner befasst sich die Erfindung mit einem Set aus derartigen Herzklappenprothesen sowie einem System mit einer solchen Herzklappenprothese oder einem derartigen Set.

Aus medizinischer Sicht stellt die operative Behandlung der Trikuspidalklappeninsuffizienz eine hohe Herausforderung dar. Dabei lässt die Dichtigkeit der Trikuspidalklappe, die den rechten Vorhof bzw. das rechte Atrium des menschlichen Herzens von der rechten Herzkammer bzw. dem rechten Ventrikel trennt, nach. Dies kann dazu führen, dass aufgrund der Kontraktion des rechten Ventrikels Blutvolumen in die obere und/oder untere Hohlvene zurückgedrängt wird. Dies führt schlussendlich zu einer Volumenbelastung des rechten Herzens sowie zu einer Druckerhöhung im venösen Kreislaufsystem. In fortgeschrittenen Stadien kann es zu einem Rechtsherzversagen mit Leberstauung und Ödembildung in peripheren Blutgefäßen kommen.

Je nach Schweregrad der Erkrankung ist es erforderlich, die natürliche Trikuspidalklappe vollständig durch eine künstliche Herzklappe zu ersetzen. Derartige chirurgische Eingriffe sind äußerst aufwändig und führen zu einer hohen Belastung des Patienten. Das bisherige Operationsverfahren wird am offenen Herzen durchgeführt, so dass der Patient während der Operation an eine Herz-Lungen-Maschine angeschlossen werden muss. Dies erhöht die Komplexität des Eingriffs und erhöht die Kreislaufbelastung für den Patienten. Entsprechend hoch ist die Mortalität bei solchen Eingriffen. Es besteht daher ein Bedürfnis nach einer minimalinvasiven Möglichkeit zur Therapie der Trikuspidalklappeninsuffizienz.

Weitere Herzklappenprothesen, die zum Stand der Technik gehören, werden in den Druckschriften DE 10 2010 046 459 A1, US 2006/0276813 A1, US 2011/0106244 A1, US 2004/0206363 A1, und WO 2014/086871 A2 beschrieben.

Die Aufgabe der Erfindung besteht darin, eine Herzklappenprothese anzugeben, die zum perkutanen Ersatz einer Trikuspidalklappe geeignet ist und insofern eine minimalinvasive operative Therapiemöglichkeit für die Trikuspidalklappeninsuffizienz bietet. Ferner besteht die Aufgabe der Erfindung darin, ein Set und ein System mit einer solchen Herzklappenprothese anzugeben.

Erfindungsgemäß wird diese Aufgabe im Hinblick auf die Herzklappenprothese durch den Gegenstand des Patentanspruchs 1, im Hinblick auf das Set durch den Gegenstand des Patentanspruchs 11 und im Hinblick auf das System durch den Gegenstand des Patentanspruchs 13 gelöst.

Die Erfindung beruht auf dem Gedanken, eine Herzklappenprothese zum perkutanen Ersatz einer Trikuspidalklappe anzugeben, wobei die Herzklappenprothese eine stentartige Tragstruktur und eine biologische Herzklappe aufweist. Die stentartige Tragstruktur ist von einem radial komprimierten in einen radial expandierten Zustand selbsttätig expandierbar und weist im radial expandierten Zustand eine Verjüngung auf. Die Verjüngung teilt die Tragstruktur in einen Hohlvenenabschnitt und in einen Atriumabschnitt, wobei die Herzklappe im Atriumabschnitt angeordnet ist, wobei der Hohlvenenabschnitt ein ringförmiges Durchlasssegment und ein Stabilisierungssegment aufweist, wobei das ringförmige Durchlasssegment im Bereich der Verjüngung angeordnet ist, wobei die stentartige Tragstruktur miteinander verflochtene Drähte oder einstückig miteinander verbundene Stege aufweist, die Maschen begrenzen, und wobei die Maschen im Durchlasssegment eine größere Maschenweite als im Stabilisierungssegment aufweisen.

Die erfindungsgemäße Herzklappenprothese ermöglicht einen heterotopen, kathetergeführten Trikuspidalklappenersatz. Insbesondere eignet sich die Herzklappenprothese für eine minimalinvasive chirurgische Implantation und reduziert somit die Belastungen des Patienten während einer Operation. So kann die erfindungsgemäße Herzklappenprothese beispielsweise transluminal über die Femoralvene an den Implantationsort geführt und dort freigesetzt werden. Dabei expandiert die Herzklappenprothese selbsttätig und spannt sich am gewünschten Implantationsort auf. Durch die selbstexpandierenden Eigenschaften wird gleichzeitig ein ausreichend hoher Radialdruck auf die Gefäßwand der Hohlvene am Implantationort ausgeübt, so dass die erfindungsgemäße Herzklappenprothese selbstdichtend mit der Hohlvene abschließt. Insofern ist die Herzklappenprothese selbstdichtend implantierbar bzw. selbstdichtend ausgebildet.

Die in der stentartigen Tragstruktur angeordnete Verjüngung bildet eine Positionierhilfe, so dass die Herzklappenprothese bei der Expansion selbsttätig an die korrekte Position gerückt wird. Vorzugsweise wird die Herzklappenprothese im Bereich des Übergangs von der oberen und/oder unteren Hohlvene zum rechten Vorhof bzw. Atrium eingesetzt. Dabei ist vorgesehen, dass der Hohlvenenabschnitt innerhalb der Hohlvene und der Atriumabschnitt innerhalb des rechten Vorhofs anordnbar sind. Insofern ist in bevorzugten Ausführungsformen der Erfindung vorgesehen, dass der Hohlvenenabschnitt zur Implantation in die obere und/oder untere Hohlvene, und der Atriumabschnitt zur Positionierung innerhalb des rechten Vorhofs angepasst sind. Der Hohlvenenabschnitt dient dabei vorzugsweise der Verankerung der Herzklappenprothese und stabilisiert insofern die Position der Herzklappenprothese am Implantationsort. Der Atriumabschnitt umfasst die biologische Herzklappe, wobei die biologische Herzklappe derart im Atriumabschnitt angeordnet sein kann, dass die biologische Herzklappe mit der oberen und/oder unteren Hohlvene abdichtet. Dies kann beispielsweise dadurch gewährleistet sein, dass die biologische Herzklappe unmittelbar an der Gefäßwand der Hohlvene im Übergangsbereich zwischen der Hohlvene und dem rechten Vorhof anliegt und/oder ein Teilbereich der stentartigen Tragstruktur, insbesondere ein Abschnitt, der wenigstens teilweise die Verjüngung und den Atriumabschnitt umfasst, mit einer fluiddichten Wandung versehen ist. Mit anderen Worten können bei bevorzugten Ausgestaltungen der Erfindung der Atriumabschnitt und/oder die Verjüngung zumindest teilweise eine fluiddichte Wandung aufweisen.

In weiteren bevorzugten Ausführungsformen der Erfindung ist vorgesehen, dass die Tragstruktur eine sanduhrförmige oder stundenglasförmige oder trichterförmige oder tulpenförmige oder pyramidenförmige Längsschnittgeometrie aufweist. Eine sanduhrförmige oder stundenglasförmige Längsschnittgeometrie kann beispielsweise durch einen Hohlvenenabschnitt und einen Atriumabschnitt gebildet werden, die jeweils kegelstumpfförmig gestaltet sind, wobei die kegelstumpfförmigen Abschnitte derart miteinander verbunden sind, dass einerseits zwischen den beiden kegelstumpfförmigen Abschnitten die Verjüngung gebildet ist und andererseits sich insgesamt eine Doppelkegelform ergibt. Alternativ ist es möglich, dass die Tragstruktur insgesamt eine trichterförmige Längsschnittgeometrie aufweist, wobei sich die Tragstruktur von einem Längsende zum gegenüberliegenden Längsende aufweitet. Die Verjüngung kann dann als Einschnitt ausgebildet sein, der die trichterförmige Geometrie der Tragstruktur bereichsweise unterbricht. Mit anderen Worten setzt sich die trichterförmige Struktur beidseitig der Verjüngung jeweils fort, so dass sich insgesamt eine im Wesentlichen trichterförmige Außengeometrie der Tragstruktur zeigt. Vorzugsweise weist der Hohlvenenabschnitt der Tragstruktur zumindest am längsaxialen, offenen Ende der Tragstruktur einen größeren Querschnittsdurchmesser als der Atriumabschnitt auf. Damit ist sichergestellt, dass im Bereich des Hohlvenenabschnitts eine ausreichend große Radialkraft wirksam ist, um die Herzklappenprothese sicher in der Hohlvene zu verankern.

In diesem Zusammenhang wird darauf hingewiesen, dass in der vorliegenden Anmeldung die Form der Herzklappenprothese, insbesondere der stentartigen Tragstruktur, im vollständig expandierten Zustand beschrieben wird. Der vollständig expandierte Zustand zeichnet sich dadurch aus, dass keine äußeren Kräfte auf die Tragstruktur wirken, die beispielsweise die Tragstruktur radial komprimieren. Im implantierten Zustand am Behandlungsort nimmt die Herzklappenprothese bzw. die stentartige Tragstruktur ggf. eine völlig andere Form ein, da die radiale Expansion durch den Kontakt mit Gefäßwänden der Hohlvene beispielsweise begrenzt sein kann.

Wie zuvor bereits erläutert wurde, können der Atriumabschnitt und/oder die Verjüngung zumindest teilweise eine fluiddichte Wandung aufweisen. Alternativ ist es auch möglich, dass die gesamte stentartige Tragstruktur mit einer fluiddichten Wandung bzw. Umhüllung versehen ist. Insofern kann die stentartige Tragstruktur als Stent-Graft ausgebildet sein, wobei der Graft-Teil bzw. die fluiddichte Wandung sowohl auf einem Innenumfang, als auch auf einem Außenumfang der Tragstruktur angeordnet sein kann. Ferner ist es möglich, dass sowohl auf dem Innenumfang, als auch auf dem Außenumfang jeweils eine fluiddichte Wandung angeordnet ist. Die stentartige Tragstruktur kann sich also zwischen zwei fluiddichten Wandungen erstrecken.

Um eine sichere Verankerung der Herzklappenprothese in der Hohlvene zu gewährleisten, kann vorgesehen sein, dass der Hohlvenenabschnitt und der Atriumabschnitt unterschiedliche Längen aufweisen. Insbesondere kann der Hohlvenenabschnitt eine Länge aufweisen, die größer als die Länge des Atriumabschnitts ist. So wird eine vergleichsweise große Kontaktfläche zwischen der Herzklappenprothese und der Gefäßwand der Hohlvene bereitgestellt, wodurch die Fixierung der Herzklappenprothese im implantierten Zustand verbessert ist.

Ferner kann vorgesehen sein, dass der Hohlvenenabschnitt und der Atriumabschnitt unterschiedliche Längsschnitt- und/oder Querschnittgeometrien aufweisen. Dabei sind die Längsschnitt- und Querschnittgeometrie des Hohlvenenabschnitts und des Atriumabschnitts jeweils derart gestaltet, dass die Herzklappenprothese sich anatomisch gut an den Implantationsort in der oberen oder unteren Hohlvene anpasst. Eine entsprechende, beispielsweise patientenindividuelle, Gestaltung der Herzklappenprothese reduziert das Thromboserisiko. So kann beispielsweise vorgesehen sein, die Längsschnitt- und/oder Querschnittgeometrie der stentartigen Tragstruktur anhand von Daten aus bildgebenden Verfahren, beispielsweise aus Computertomografie-Daten und/oder Ultraschalldiagnostik-Daten und/oder Magnetresonanztomografie-Daten, zu ermitteln.

Die stentartige Tragstruktur kann im Allgemeinen miteinander verflochtene Drähte oder einstückig miteinander verbundene Stege aufweisen, die jeweils Maschen begrenzen. Vorzugsweise umfasst die stentartige Tragstruktur eine geschlossene Maschenstruktur bzw. ist nach Art eines Closed-Cell-Designs ausgebildet. Dabei kann in bevorzugten Varianten vorgesehen sein, dass die Maschen der Tragstruktur bereichsweise unterschiedliche Maschenweiten aufweisen. Mit anderen Worten kann die Größe der einzelnen Maschen bzw. deren Durchlassfläche, unterschiedlich sein.

Im Allgemeinen können die Maschen im Wesentlichen rautenförmig ausgebildet sein. Jede einzelne Masche ist also durch vier Drähte oder Stege begrenzt. Zur Anpassung der Maschenweite kann der Winkel zwischen den einzelnen Drähten bzw. Stegen und der Längsachse der stentartigen Tragstruktur variiert werden. So können entlang der stentartigen Tragstruktur ringförmige Abschnitte gebildet sein, die Maschen aufweisen, deren Maschenweite größer als die Maschenweite von Maschen benachbarter ringförmiger Segmente ist.

Insbesondere kann der Hohlvenenabschnitt der stentartigen Tragstruktur ein Durchlasssegment und ein Stabilisierungssegment aufweisen. Das Durchlasssegment und das Stabilisierungssegment können jeweils ringförmige Segmente des Hohlvenenabschnitts bilden. Die ringförmigen Segmente umfassen jeweils eine Vielzahl von Maschen, die in Umfangsrichtung der Tragstruktur benachbart zueinander angeordnet sind. Die Maschen im Durchlasssegment weisen vorzugsweise eine größere Maschenweite auf, als die Maschen im Stabilisierungssegment. Insofern kann das Drahtgeflecht bzw. die Gitterstruktur, die die stentartige Tragstruktur bildet, im Stabilisierungssegment eine höhere Maschendichte aufweisen. Folglich bietet das Stabilisierungssegment eine größere Kontaktoberfläche zur Anlage an die Gefäßwand der Hohlvene und verbessert so die Fixierung der Herzklappenprothese. Das Durchlasssegment ermöglicht hingegen eine gute Durchströmung der stentartigen Tragstruktur, so dass durch die Tragstruktur strömendes Blut beispielsweise seitlich aus dem Hohlvenenabschnitt ausströmen kann. Insofern ist bevorzugt vorgesehen, dass das Durchlasssegment frei von einer fluiddichten Wandung ist.

Vorzugsweise ist das Durchlasssegment im implantierten Zustand der Herzklappenprothese innerhalb der Hohlvene in einem Bereich mit einmündenden Blutgefäßen positionierbar. Bei der Implantation der Herzklappenprothese in die untere Hohlvene erstreckt sich der Hohlvenenabschnitt vorzugsweise über den Einstrombereich der Lebervenen hinweg. Das Durchlasssegment des Hohlvenenabschnitts ist daher vorzugsweise in einem Bereich angeordnet, der im implantierten Zustand im Bereich des Lebervenen-Einstroms positionierbar ist. So ist verhindert, dass der Bluteinstrom von den Lebervenen in die untere Hohlvene blockiert ist.

In einer bevorzugten Ausführungsform der vorliegenden Erfindung ist vorgesehen, dass die Tragstruktur ein Formgedächtnismaterial und/oder ein superelastisches Material, insbesondere eine Nickel-Titan-Legierung, vorzugsweise Nitinol, aufweist oder daraus gebildet ist. Derartige Formgedächtnismaterialien ermöglichen eine besonders gute selbstexpandierende Funktion und weisen die notwendige Biokompatibilität zur Implantation in Blutgefäßen auf. Ferner umfassen derartige Materialien eine ausreichend hohe Stabilität, so dass die stentartige Tragstruktur vergleichsweise dünn ausgebildet werden kann. Schließlich ermöglichen insbesondere Nickel-Titan-Legierungen eine gute Komprimierung der Tragstruktur, was die Implantation über einen Katheter erleichtert.

Im Allgemeinen kann die Herzklappenprothese transluminal, insbesondere transfemoral, implantierbar sein. Dies gewährleistet einen minimalinvasiven Eingriff und reduziert die Belastung des Patienten während der Operation. So kann die Herzklappenprothese über einen Katheter an den Implantationsort geführt werden, wobei der Katheter vorzugsweise über die Leistenvene bzw. Femoralvene und die untere Hohlvene zum rechten Herzvorhof vorgeschoben wird. Im Bereich des Übergangs zwischen der unteren Hohlvene und dem rechten Herzvorhof wird die Herzklappenprothese aus dem Katheter entlassen und spannt sich selbsttätig auf. Dabei gelangt der Hohlvenenabschnitt in Anlage mit der Gefäßwand der unteren Hohlvene. Der Atriumabschnitt ragt in den rechten Vorhof vor. Die im Atriumabschnitt angeordnete Herzklappe spannt sich dabei auf und übernimmt, zumindest teilweise, die Funktion der Trikuspidalklappe. Für die Implantation der Herzklappenprothese im Bereich der oberen Hohlvene kann auch ein Zugang über die Schlüsselbeinvene (vena subclavia) gewählt werden. Alternativ kann auch ein Femoralvenenkatheter über den rechten Vorhof in die obere Hohlvene geführt werden, so dass die Herzklappenprothese in der oberen Hohlvene freisetzbar ist.

Die biologische Herzklappe der erfindungsgemäßen Herzklappenprothese ist vorzugsweise fest im Atriumabschnitt fixiert. Dabei ist vorgesehen, dass die biologische Herzklappe derart flexibel ist, dass sie sich einer Komprimierung der stentartigen Tragstruktur anpasst und bei der selbsttätigen Expansion am Implantationsort ebenfalls entfaltet wird. Vorzugsweise ist die biologische Herzklappe derart stabil im Atriumabschnitt fixiert, dass die biologische Herzklappe nach der Expansion am Implantationsort unmittelbar wirksam ist und die Funktion der Trikuspidalklappe zumindest teilweise übernimmt.

Die Erfindung umfasst außerdem ein Set aus zwei zuvor beschriebenen Herzklappenprothesen, wobei eine erste Herzklappenprothese zur Verankerung in der oberen Hohlvene und eine zweite Herzklappenprothese zur Verankerung in der unteren Hohlvene angepasst sind.

Das vorgeschlagene Set ermöglicht den vollständigen Ersatz der Funktion der natürlichen Trikuspidalklappe. Um diese Funktion minimalinvasiv chirurgisch zu reproduzieren, wird vorgeschlagen, zwei Herzklappenprothesen einzusetzen, die jeweils in der oberen und unteren Hohlvene angeordnet sind. Die natürliche Trikuspidalklappe kann dabei erhalten werden, wird jedoch durch die Herzklappenprothesen unterstützt. Mithin wird dadurch die Funktion der Trikuspidalklappe (zumindest teilweise) ersetzt. So kann eine Funktionsbeeinträchtigung der natürlichen Trikuspidalklappe kompensiert werden.

Mit anderen Worten, wird eine mangelhafte Dichtigkeit der natürlichen Trikuspidalklappe dadurch kompensiert, dass der natürlichen Trikuspidalklappe zwei biologische Herzklappenprothesen vorgeschaltet werden, die gegenüber den Hohlvenen ausreichend abdichten. Dies senkt die Volumenbelastung des rechten Herzens und die Druckerhöhung im venösen System.

Die beiden Herzklappenprothesen können unterschiedliche Längsschnittgeometrien und/oder Querschnittgeometrien aufweisen. Grundsätzlich können sich die beiden Herzklappenprothesen in ihrer Formgebung voneinander unterscheiden, wobei die beiden Herzklappenprothesen jeweils an ihren Implantationsort angepasst sind. So kann die erste Herzklappenprothese eine andere geometrische Struktur als die zweite Herzklappenprothese aufweisen. Beispielsweise kann die erste Herzklappenprothese im Wesentlichen trichterförmig und die zweite Herzklappenprothese sanduhrförmig ausgebildet sein. Es ist auch möglich, dass die zweite Herzklappenprothese, die in der unteren Hohlvene implantiert wird, ein Durchlasssegment aufweist, um den Bluteinstrom aus den Lebervenen in die untere Hohlvene zu gewährleisten. Die erste Herzklappenprothese kann hingegen eine Tragstruktur mit einer gleichmäßigen Maschenweite aufweisen.

Im Rahmen der vorliegenden Erfindung wird außerdem ein System mit einer zuvor beschriebenen Herzklappenprothese oder einem zuvor beschriebenen Set und einem Katheter offenbart und beansprucht. Dabei ist (sind) die Herzklappenprothese(n) in einem komprimierten Zustand innerhalb des Katheters positionierbar und mittels des Katheters perkutan, insbesondere transluminal, implantierbar. In Verbindung mit dem Set aus zwei Herzklappenprothesen kann auch vorgesehen sein, dass das System zwei Katheter aufweist, wobei ein erster Katheter der ersten Herzklappenprothese und ein zweiter Katheter der zweiten Herzklappenprothese zugeordnet sind.

Die Erfindung wird im Folgenden anhand eines Ausführungsbeispiels unter Bezugnahme auf die beigefügte Zeichnung näher erläutert. Darin zeigt die einzige Figur ein Set aus zwei Herzklappenprothesen nach einem bevorzugten Ausführungsbeispiel der Erfindung im implantierten Zustand.

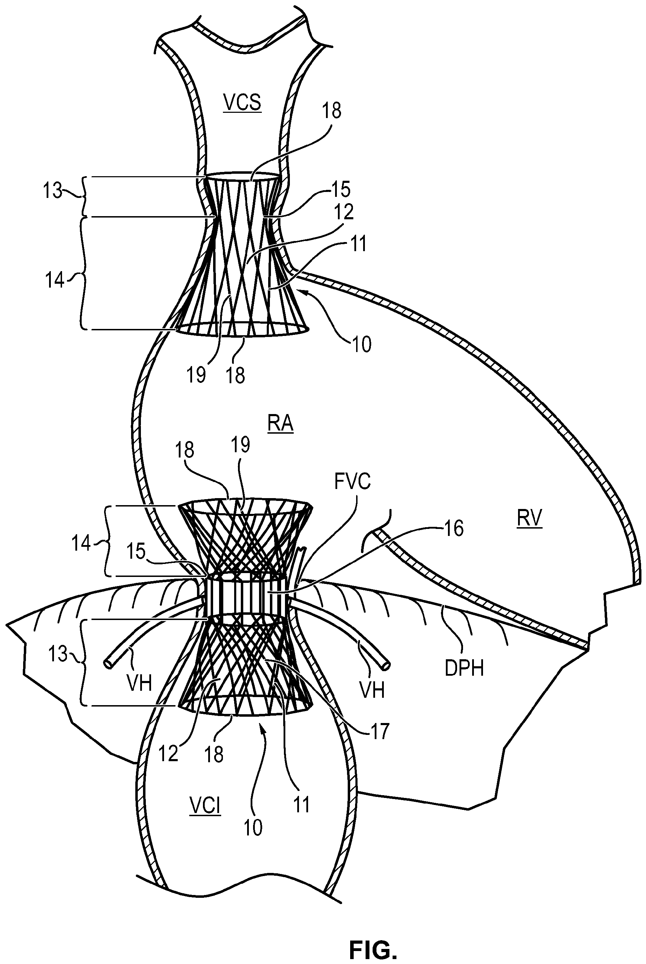

Fig. 1 zeigt einen Schnitt durch das rechte Herz, das sich in einen rechten Vorhof RA und eine Herzkammer RV teilt. Zwischen der rechten Herzkammer RV und dem rechten Vorhof RA ist die natürliche Trikuspidalklappe (nicht dargestellt) angeordnet. Um eine Insuffizienz der natürlichen Trikuspidalklappe zu kompensieren, ist ein Set aus zwei Herzklappenprothesen 10 vorgesehen, die im Übergangsbereich von der oberen Hohlvene VCS in den rechten Vorhof RA sowie im Übergangsbereich von der unteren Hohlvene VCI in dem rechten Vorhof RA angeordnet sind.

Die beiden Herzklappenprothesen 10 weisen jeweils eine Tragstruktur 11 auf, die einen Hohlvenenabschnitt 13 und einen Atriumabschnitt 14 umfasst. Der Hohlvenenabschnitt und der Atriumabschnitt 14 sind vorzugsweise durch einen Verjüngung 15 voneinander getrennt. Mit anderen Worten weist die Tragstruktur 11 an ihren längsaxialen Enden 18 jeweils einen größeren Querschnittsdurchmesser als im Bereich der Verjüngung 15 auf. Die Tragstruktur 11 umfasst mehrere Maschen 12, die durch ein Geflecht von sich kreuzenden Drähten 19 gebildet sind. Alternativ zu dem Drahtgeflecht, das in der Figur dargestellt ist, können auch einstückige Tragstrukturen 11 aus einteilig miteinander verbundenen Stegen vorgesehen sein. Derartige Tragstrukturen sind beispielsweise durch Laserschneiden aus einem rohrförmigen Rohmaterial herstellbar. Jedenfalls ist bei dem dargestellten Ausführungsbeispiel vorgesehen, dass die Tragstruktur 11 eine Gitterstruktur bzw. ein Geflecht aus geschlossenen Zellen bzw. Maschen aufweist (Closed-Cell Design).

Die beiden Herzklappenprothesen 10 gemäß der beigefügten Figur weisen jeweils eine Tragstruktur 11 mit einer sanduhrförmigen Längsschnittgeometrie auf. Mit anderen Worten sind der Hohlvenenabschnitt 13 und der Atriumabschnitt 14 jeweils kegelstumpfförmig ausgebildet, wobei die beiden Kegelstümpfe derart aufeinander stehen, dass zwischen den beiden Kegelstumpfformen die Verjüngung 15 ausgebildet ist. Wie aus der Figur erkennbar ist, ist der Hohlvenenabschnitt 13 jedenfalls in der oberen Hohlvene VCS bzw. unteren Hohlvene VCI angeordnet. Der Atriumabschnitt 14 erstreckt sich zumindest teilweise in den rechten Vorhof RA hinein. Die Herzklappenprothese 10 umfasst ferner eine nicht dargestellte Herzklappe, die vorzugsweise als biologische Herzklappe ausgebildet ist. Die Herzklappe ist im Atriumabschnitt 14 angeordnet und dort fest fixiert.

Eine besondere Herausforderung stellt sich für die Herzklappenprothese 10, die im Bereich der unteren Hohlvene VCI implantiert ist. Denn die untere Hohlvene VCI erstreckt sich durch eine Zwerchfellöffnung FVC, die im Diaphragma bzw. Zwerchfell DPH angeordnet ist. Das Zwerchfell DPH unterliegt starken Bewegungen, die sich auch auf die untere Hohlvene VCI und folglich auf die darin angeordnete Herzklappenprothese 10 auswirkt. Die Tragstruktur 11 ist daher ausgeprägt sanduhrförmig ausgebildet, um eine stabile Verankerung am Implantationsort zu gewährleisten. Die ausgeprägte sanduhrförmige Längsschnittgeometrie verhindert eine Deplatzierung der Herzklappenprothese 10 infolge der Bewegung des Zwerchfells DPH.

Eine weitere anatomische Schwierigkeit ergibt sich bei der Implantation der Herzklappenprothese 10 in der unteren Hohlvene VCI durch die Blut zuführenden Lebervenen VH, die im Bereich der Zwerchfellöffnung FVC in die untere Hohlvene VCI münden. Um zu verhindern, dass die Tragstruktur 11 aufgrund ihrer Maschendichte den Zufluss von venösem Blut aus den Lebervenen VH in die untere Hohlvene VCI blockieren, ist vorgesehen, dass der Hohlvenenabschnitt 13 ein Durchlasssegment 16 aufweist. Das Durchlasssegment 16 unterscheidet sich von einem Stabilisierungssegment 17 des Hohlvenenabschnitts 13 durch eine unterschiedliche Maschendichte bzw. Maschenweite. So sind die Maschen des Durchlasssegments 16 vorzugsweise größer ausgebildet als die Maschen des Stabilisierungssegments 17. Die erhöhte Maschenweite im Durchlasssegment 16 verbessert die Durchlässigkeit der Tragstruktur 11 für seitlich einströmendes Blut und gewährleistet so einen guten Einstrom von venösem Blut aus der Leber in die untere Hohlvene VCI. Dies reduziert das Risiko eines erhöhten venösen Drucks in den Lebervenen VH.

Wie in der Figur gut erkennbar ist, ist die Herzklappenprothese 10 zur Anordnung in der unteren Hohlvene VCI im Wesentlichen symmetrisch gebildet, wobei die Symmetrie durch die Verjüngung 15 gebildet wird. Zumindest das Stabilisierungssegment 17 des Hohlvenenabschnitts 13 ist im Wesentlichen analog zum Atriumabschnitt 14 ausgebildet. Der Atriumabschnitt 14 umfasst eine kegelstumpfförmige Geometrie. Hinsichtlich der Längendimensionen können der Atriumabschnitt 14 und der Hohlvenenabschnitt 13, insbesondere das Stabilisierungssegment 17, unterschiedlich ausgebildet sein. Vorzugsweise ist der Hohlvenenabschnitt 13 länger als der Atriumabschnitt 14.

Bei der Herzklappenprothese 10 zur Anordnung in der oberen Hohlvene VCS ist eine andere Gestaltung bevorzugt. Der Hohlvenenabschnitt 13 ist dabei kürzer ausgebildet als der Atriumabschnitt 14. Allerdings erstreckt sich auch der Atriumabschnitt 14 zumindest teilweise in die obere Hohlvene VCS hinein und übernimmt somit ebenfalls eine Verankerungsfunktion. Der Hohlvenenabschnitt 13 weist ein längsaxiales Ende 18 auf, das einen Querschnittsdurchmesser umfasst, welcher größer als der Querschnittsdurchmesser der oberen Hohlvene VCS ist. Das bewirkt, dass die obere Hohlvene VCS im Bereich des Hohlvenenabschnitts 13 aufgeweitet bzw. expandiert wird und so die Herzklappenprothese 10 sicher fixiert ist. Insofern kann vorgesehen sein, dass der Hohlvenenabschnitt 13 ausschließlich zur Verankerung der Herzklappenprothese 10 in der oberen Hohlvene VCS oder der unteren Hohlvene VCI dient.

### Bezugszeichen

- 10: Herzklappenprothese
- 11: Tragstruktur
- 12: Masche
- 13: Hohlvenenabschnitt
- 14: Atriumabschnitt
- 15: Verjüngung
- 16: Durchlasssegment
- 17: Stabilisierungssegment
- 18: Längsaxiales Ende
- 19: Draht
- VCS: Obere Hohlvene
- VCI: Untere Hohlvene
- RA: Rechter Vorhof
- RV: Rechte Herzkammer
- DPH: Zwerchfell
- FVC: Zwerchfellöffnung
- VH: Lebervene

## Patentansprüche

1. Herzklappenprothese (10) zum perkutanen Ersatz einer Trikuspidalklappe mit einer stentartigen Tragstruktur (11) und einer biologischen Herzklappe, wobei die stentartige Tragstruktur (11) von einem radial komprimierten in einen radial expandierten Zustand selbsttätig expandierbar ist und im radial expandierten Zustand eine Verjüngung (15) aufweist, die die Tragstruktur (11) in einen Hohlvenenabschnitt (13) und in einen Atriumabschnitt (14) teilt, wobei die Herzklappe im Atriumabschnitt angeordnet ist, wobei der Hohlvenenabschnitt (13) ein ringförmiges Durchlasssegment (16) und ein Stabilisierungssegment (17) aufweist,
wobei das ringförmige Durchlasssegment (16) im Bereich der Verjüngung (15) angeordnet ist, wobei die stentartige Tragstruktur (11) miteinander verflochtene Drähte (19) oder einstückig miteinander verbundene Stege aufweist, die Maschen (12) begrenzen, **dadurch gekennzeichnet, dass** die Maschen (12) im Durchlasssegment (16) eine größere Maschenweite als im Stabilisierungssegment (17) aufweisen.

2. Herzklappenprothese (10) nach Anspruch 1,
**dadurch gekennzeichnet, dass**
der Hohlvenenabschnitt (13) zur Implantation in die obere und/oder untere Hohlvene (VCS, VCI) und der Atriumabschnitt (14) zur Positionierung innerhalb des rechten Herzvorhofs (RA) angepasst ist.

3. Herzklappenprothese (10) nach Anspruch 1 oder 2,
**dadurch gekennzeichnet, dass**
der Atriumabschnitt (14) und/oder die Verjüngung (15) zumindest teilweise eine fluiddichte Wandung aufweisen.

4. Herzklappenprothese (10) nach einem der vorhergehenden Ansprüche, **dadurch gekennzeichnet, dass** die Tragstruktur (11) eine sanduhrförmige oder stundenglasförmige oder trichterförmige oder tulpenförmige oder pyramidenförmige Längsschnittgeometrie aufweist.

5. Herzklappenprothese (10) nach einem der vorhergehenden Ansprüche,
**dadurch gekennzeichnet, dass**
der Hohlvenenabschnitt (13) und der Atriumabschnitt (14) unterschiedliche Längen aufweisen.

6. Herzklappenprothese (10) nach einem der vorhergehenden Ansprüche,
**dadurch gekennzeichnet, dass**
der Hohlvenenabschnitt (13) und der Atriumabschnitt (14) unterschiedliche Längsschnitt- und/oder Querschnittgeometrien aufweisen.

7. Herzklappenprothese (10) nach einem der vorhergehenden Ansprüche,
**dadurch gekennzeichnet, dass**
das Durchlasssegment (16) im implantierten Zustand innerhalb der Hohlvene (VCS, VCI) in einem Bereich mit einmündenden Blutgefäßen positionierbar ist.

8. Herzklappenprothese (10) nach einem der vorhergehenden Ansprüche,
**dadurch gekennzeichnet, dass**
die Tragstruktur (11) ein Formgedächtnismaterial und/oder superelastisches Material, insbesondere eine Nickel-Titan-Legierung, vorzugsweise Nitinol, aufweist oder daraus gebildet ist.

9. Herzklappenprothese (10) nach einem der vorhergehenden Ansprüche,
**dadurch gekennzeichnet, dass**
die Herzklappenprothese (10) transluminal, insbesondere transfemoral, implantierbar ist.

10. Herzklappenprothese (10) nach einem der vorhergehenden Ansprüche,
**dadurch gekennzeichnet, dass**
die biologische Herzklappe fest im Atriumabschnitt (14) fixiert ist.

11. Set aus zwei Herzklappenprothesen (10), wobei eine erste Herzklappenprothese (10) mit einer stentartigen Tragstruktur (11) und einer biologischen Herzklappe, wobei die stentartige Tragstruktur (11) von einem radial komprimierten in einen radial expandierten Zustand selbsttätig expandierbar ist und im radial expandierten Zustand eine Verjüngung (15) aufweist, die die Tragstruktur (11) in einen Hohlvenenabschnitt (13) und in einen Atriumabschnitt (14) teilt, wobei die Herzklappe im Atriumabschnitt angeordnet ist, zur Verankerung in der oberen Hohlvene (VCS) und eine zweite Herzklappenprothese (10) nach zumindest einem der vorherigen Ansprüche zur Verankerung in der unteren Hohlvene (VCI) angepasst ist.

12. Set nach Anspruch 11,
**dadurch gekennzeichnet, dass**
die Herzklappenprothesen (10) unterschiedliche Längsschnittgeometrien und/oder Querschnittgeometrien aufweisen.

13. System mit einer Herzklappenprothese (10) oder einem Set nach einem der vorhergehenden Ansprüche und einem Katheter, wobei die Herzklappenprothese(n) (10) in einem komprimierten Zustand innerhalb des Katheters positionierbar und mittels des Katheters perkutan, insbesondere transluminal, implantierbar ist/sind.

## Claims

1. A heart valve prosthesis (10) for a percutaneous replacement of a tricuspid valve, said heart valve prosthesis (10) comprising a stent-like support structure (11) and a biological heart valve, wherein the stent-like support structure (11) is automatically expandable from a radially compressed state into a radially expanded state and has a tapering (15) in the radially expanded state that divides the support structure (11) into a vena cava section (13) and an atrium section (14), wherein the heart valve is arranged in the atrium section, wherein the vena cava section (13) has an annular passage segment (16) and a stabilizing segment (17),
wherein the annular passage segment (16) is arranged in the region of the tapering (15), wherein the stent-like support structure (11) has interwoven wires (19) or webs that are connected in one piece to one another and that bound meshes (12), **characterized in that** the meshes (12) have a larger mesh width in the passage segment (16) than in the stabilizing segment (17).

2. A heart valve prosthesis (10) in accordance with claim 1,
**characterized in that**
the vena cava section (13) is adapted for implantation into the superior vena cava and/or inferior vena cava (VCS, VCI) and the atrium section (14) is adapted for positioning within the right atrium (RA).

3. A heart valve prosthesis (10) in accordance with claim 1 or claim 2,
**characterized in that**
the atrium section (14) and/or the tapering (15) at least partly has/have a fluid-tight wall.

4. A heart valve prosthesis (10) in accordance with any one of the preceding claims,
**characterized in that** the support structure (11) has a sandglass-shaped or hourglass-shaped or funnel-shaped or tulip-shaped or pyramid-shaped longitudinal section geometry.

5. A heart valve prosthesis (10) in accordance with any one of the preceding claims,
**characterized in that**
the vena cava section (13) and the atrium section (14) have different lengths.

6. A heart valve prosthesis (10) in accordance with any one of the preceding claims,
**characterized in that**
the vena cava section (13) and the atrium section (14) have different longitudinal section geometries and/or cross-sectional geometries.

7. A heart valve prosthesis (10) in accordance with any one of the preceding claims,
**characterized in that**,
in the implanted state, the passage segment (16) can be positioned within the vena cava (VCS, VCI) in a region with blood vessels opening into it.

8. A heart valve prosthesis (10) in accordance with any one of the preceding claims,
**characterized in that**
the support structure (11) has or is formed from a shape memory material and/or a superelastic material, in particular a nickel-titanium alloy, preferably nitinol.

9. A heart valve prosthesis (10) in accordance with any one of the preceding claims,
**characterized in that**
the heart valve prosthesis (10) can be transluminally, in particular transfemorally, implanted.

10. A heart valve prosthesis (10) in accordance with any one of the preceding claims,
**characterized in that**
the biological heart valve is firmly fixed in the atrium section (14).

11. A set of two heart valve prostheses (10), wherein a first heart valve prosthesis (10) comprises a stent-like support structure (11) and a biological heart valve, wherein the stent-like support structure (11) is automatically expandable from a radially compressed state into a radially expanded state and has a tapering (15) in the radially expanded state that divides the support structure (11) into a vena cava section (13) and an atrium section (14), wherein the heart valve is arranged in the atrium section and is adapted for anchoring in the superior vena cava (VCS) and a second heart valve prosthesis (10) in accordance with at least one of the preceding claims is adapted for anchoring in the inferior vena cava (VCI).

12. A set in accordance with claim 11,
**characterized in that**
the heart valve prostheses (10) have different longitudinal section geometries and/or cross-sectional geometries.

13. A system comprising a heart valve prosthesis (10) or a set in accordance with any one of the preceding claims and a catheter, wherein, in a compressed state, the heart valve prosthesis/prostheses (10) can be positioned within the catheter and can be percutaneously, in particular transluminally, implanted by means of the catheter.

## Revendications

1. Prothèse de valvule cardiaque (10) pour le remplacement percutané d'une valvule tricuspide, comprenant une structure de support de type stent (11) et une valvule cardiaque biologique,
dans laquelle
la structure de support de type stent (11) est auto-expansible d'un état radialement comprimé à un état radialement expansé et présente, dans l'état radialement expansé, un rétrécissement (15) qui subdivise la structure de support (11) en une portion veine cave (13) et en une portion atrium (14), la valvule cardiaque est disposée dans la portion atrium,
la portion veine cave (13) comprend un segment de passage annulaire (16) et un segment de stabilisation (17),
le segment de passage annulaire (16) est disposé au niveau du rétrécissement (15),
la structure de support de type stent (11) comprend des fils entrelacés (19) ou des barrettes interconnectées d'un seul tenant, qui délimitent des mailles (12),
**caractérisée en ce que**
les mailles (12) dans le segment de passage (16) ont une taille de maillage supérieure à celle dans le segment de stabilisation (17).

2. Prothèse de valvule cardiaque (10) selon la revendication 1,
**caractérisée en ce que**
la portion veine cave (13) est adaptée pour être implantée dans la veine cave supérieure et/ou inférieure (VCS, VCI), et la portion atrium (14) est adaptée pour être positionnée à l'intérieur de l'oreillette droite (RA).

3. Prothèse de valvule cardiaque (10) selon la revendication 1 ou 2,
**caractérisée en ce que**
la portion atrium (14) et/ou le rétrécissement (15) comprennent au moins partiellement une paroi étanche aux fluides.

4. Prothèse de valvule cardiaque (10) selon l'une des revendications précédentes,
**caractérisée en ce que**
la structure de support (11) présente une géométrie de section longitudinale en forme de sablier ou en forme d'entonnoir ou en forme de tulipe ou en forme de pyramide.

5. Prothèse de valvule cardiaque (10) selon l'une des revendications précédentes,
**caractérisée en ce que**
la portion veine cave (13) et la portion atrium (14) présentent des longueurs différentes.

6. Prothèse de valvule cardiaque (10) selon l'une des revendications précédentes,
**caractérisée en ce que**
la portion veine cave (13) et la portion atrium (14) présentent des géométries différentes en section longitudinale et/ou en section transversale.

7. Prothèse de valvule cardiaque (10) selon l'une des revendications précédentes,
**caractérisée en ce que**
à l'état implanté, le segment de passage (16) peut être positionné à l'intérieur de la veine cave (VCS, VCI) dans une zone où des vaisseaux sanguins y affluent.

8. Prothèse de valvule cardiaque (10) selon l'une des revendications précédentes,
**caractérisée en ce que**
la structure de support (11) comprend ou est formée d'un matériau à mémoire de forme et/ou d'un matériau super-élastique, en particulier d'un alliage nickel-titane, de préférence de nitinol.

9. Prothèse de valvule cardiaque (10) selon l'une des revendications précédentes,
**caractérisée en ce que**
la prothèse de valvule cardiaque (10) peut être implantée par voie transluminale, en particulier par voie transfémorale.

10. Prothèse de valvule cardiaque (10) selon l'une des revendications précédentes,
**caractérisée en ce que**
la valvule cardiaque biologique est fermement fixée dans la portion atrium (14).

11. Ensemble de deux prothèses de valvule cardiaque (10),
dans lequel
une première prothèse de valvule cardiaque (10) comprenant une structure de support de type stent (11) et une valvule cardiaque biologique est adaptée pour être ancrée dans la veine cave supérieure (VCS), la structure de support de type stent (11) étant auto-expansible d'un état radialement comprimé à un état radialement expansé et présentant, dans l'état radialement expansé, un rétrécissement (15) qui subdivise la structure de support (11) en une portion veine cave (13) et en une portion atrium (14), la valvule cardiaque étant disposée dans la portion atrium, et
une seconde prothèse de valvule cardiaque (10) selon l'une au moins des revendications précédentes est adaptée pour être ancrée dans la veine cave inférieure (VCI).

12. Ensemble selon la revendication 11,
**caractérisé en ce que**
les prothèses de valvule cardiaque(10) présentent différentes géométries de section longitudinale et/ou différentes géométries de section transversale.

13. Système comprenant une prothèse de valvule cardiaque (10) ou un ensemble selon l'une des revendications précédentes et un cathéter, dans lequel
à un état comprimé, la ou les prothèses de valvule cardiaque (10) peuvent être positionnées à l'intérieur du cathéter et être implantées par voie percutanée, en particulier par voie transluminale, au moyen du cathéter.
